# EUROPEAN PATENT APPLICATION

(11) **EP 4 495 256 A1**
(43) Date of publication of application: **22.01.2025**
(21) Application number: 24787400.1
(22) Date of filing: 19.07.2024
(51) Int. Cl.: C12Q 1/6869, C12Q 1/6874, C12N 15/11, C12Q 1/6834

(54) **SINGLE MOLECULE SEQUENCING METHOD**

(30) Priority: 30.05.2023 CN 202310632148
(71) Applicant: GeneMind Biosciences Company Limited, Shenzhen, Guangdong 518023 (CN)
(72) Inventor: CHEN, Meirong, Shenzhen, Guangdong 518023 (CN); LIU, Bin, Shenzhen, Guangdong 518023 (CN); JIN, Huan, Shenzhen, Guangdong 518023 (CN); CHEN, Fang, Shenzhen, Guangdong 518023 (CN); SUN, Lei, Shenzhen, Guangdong 518023 (CN)
(74) Representative: Schiweck Weinzierl Koch Patentanwälte Partnerschaft mbB
(86) International application number: PCT/CN2024/106448
(87) International publication number: WO 2024/245460

(57) **Abstract**

The present disclosure relates to the field of sequencing, and in particular to, a single-molecule sequencing method. The present disclosure provides the single-molecule sequencing method, comprising the following steps: (i) providing a solid carrier comprising a plurality of sites, each of the sites having a nucleic acid template-primer complex immobilized thereon; (ii) binding four types of nucleotides or analogs thereof carrying different optically detectable labels to the nucleic acid template-primer complex by a polymerization reaction to achieve single-base extension and obtain an extension product; (iii) exciting the optically detectable label to generate an optical signal, and imaging the extension product to obtain an image; (iv) removing the optically detectable label on the extension product; (v) (iii) replacing the nucleic acid template-primer complex with the extension product from which the optically detectable label is cleaved, and repeating (ii) to (iv) above one or more times to determine a template nucleic acid sequence. The single-molecule sequencing method provided by the present disclosure is beneficial to reducing the error rate, improving the sequencing quality, and shortening the sequencing time.

## Description

### TECHNICAL FIELD

The present disclosure relates to the field of biotechnology, and relates to a single-molecule sequencing method.

### BACKGROUND

Compared with next-generation high-throughput sequencing (NGS), single-molecule sequencing is the direct sequencing of a target nucleic acid molecule linked to a tag after being immobilized on the surface of a solid carrier, and PCR amplification of the target nucleic acid molecule is not needed, thereby simplifying the construction process of a sequencing library, and avoiding errors caused by the amplification of nucleic acid molecules.

During single-molecule sequencing, according to the number of the types of nucleotides or analogs thereof added in each polymerization reaction, the single-molecule sequencing can be divided into one-color sequencing and multi-color sequencing, and the multi-color sequencing can also be divided into two-color sequencing and four-color sequencing. For the one-color sequencing, four single-base extensions are performed by sequentially adding 4 types of optically detectable labeled nucleotides (G, A, T, and C); in each extension reaction, a signal generated by an optically detectable label for a certain field of view or certain fields of view is acquired, and the type of nucleotide introduced at each site is determined from the signal generated at this site; and then, the next cycle of reaction is performed by removing the optically detectable label. Thus, the process is repeated multiple cycles to obtain a group of corresponding signal images for the same field of view at different moments (each cycle of reaction), and then the information based on this group of images is superimposed according to the order of the reactions to determine the sequence of the target nucleic acid molecule. Unlike the one-color sequencing, in the multi-color sequencing, multiple types, e.g., two types or four types, of nucleotides are simultaneously added at each single-base extension, i.e., at least two types of nucleotides are introduced at each single-base extension. For example, in the two-color sequencing, two types of nucleotides can be added at each base extension, and one cycle of sequencing can be completed by two base extensions. Therefore, compared with the one-color sequencing, the whole sequencing time of the multi-color sequencing can be shortened, but the sequencing read length and the sequencing error rate still have limitations.

### SUMMARY

In view of this, the present disclosure provides a single-molecule sequencing method. The single-molecule sequencing method provided by the present disclosure is beneficial to reducing the sequencing error rate, improving the sequencing quality, and shortening the sequencing time while having a relatively long sequencing read length.

In order to achieve the above objective, the present disclosure provides the following technical schemes:
The present disclosure provides a single-molecule sequencing method, including the following steps:
(i) providing a solid carrier comprising a plurality of sites, each of the sites having a nucleic acid template-primer complex immobilized thereon;
(ii) binding four types of nucleotides or analogs thereof carrying different optically detectable labels to the nucleic acid template-primer complex by a polymerization reaction to achieve single-base extension and obtain an extension product;
(iii) exciting the optically detectable label to generate an optical signal, and imaging the extension product to obtain an image;
(iv) removing the optically detectable label on the extension product; and
(v) replacing the nucleic acid template-primer complex with the extension product from which the optically detectable label is cleaved, and repeating (ii) to (iv) above one or more times to determine a template nucleic acid sequence.

In some embodiments of the present disclosure, a mixture solution of two or four types of nucleotides or analogs thereof carrying different optically detectable labels is contacted with the nucleic acid template-primer complex to enable the nucleotides or the analogs thereof to bind to the nucleic acid template-primer complex.

In some embodiments of the present disclosure, the four types of nucleotides have a final concentration of 125-1000 nmol/L, respectively.

In some embodiments of the present disclosure, the nucleotides or the analogs thereof have a general formula shown in formula 1: 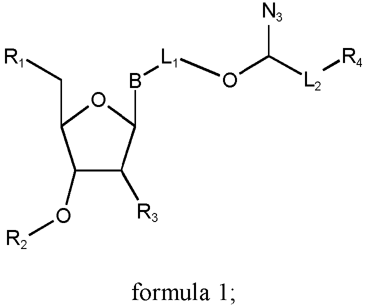 where B is a base or an analog thereof, L₁ and L₂ are each independently a covalent bond or a covalent linker group, R₁ is -OH or a phosphate group, R₂ is H or a chemically cleavable group, R₃ is H or -OR₅, R₅ is H or a chemically cleavable group, and R₄ contains the optically detectable label.

In some embodiments of the present disclosure, B is selected from adenine or an analog thereof, guanine or an analog thereof, cytosine or an analog thereof, thymine or an analog thereof, and uracil or an analog thereof.

In some embodiments of the present disclosure, in step (ii) of the sequencing method, B is selected from adenine or an analog thereof, cytosine or an analog thereof, thymine or an analog thereof, and uracil or an analog thereof, and the nucleotide or the analog thereof has a final concentration of 500-1000 nmol/L; and B is guanine or an analog thereof, and the nucleotide or the analog thereof has a final concentration of 125-500 nmol/L.

In some embodiments of the present disclosure, the optically detectable labels carried by the different nucleotides or analogs thereof are different.

In some embodiments of the present disclosure, the optically detectable label is selected from one or more of Atto532, CY5, IF700, ROX, a rhodamine dye, a cyanine dye, a coumarin dye, or a phycoerythrin.

In some embodiments of the present disclosure, when the nucleotide or the analog thereof is an adenine nucleotide or an analog thereof, the optically detectable label of the nucleotide or the analog thereof is Atto532; when the nucleotide or the analog thereof is a guanine nucleotide or an analog thereof, the optically detectable label of the nucleotide or the analog thereof is CY5; when the nucleotide or the analog thereof is a cytosine nucleotide or an analog thereof, the optically detectable label of the nucleotide or the analog thereof is IF700; when the nucleotide or the analog thereof is a thymine nucleotide or an analog thereof, the optically detectable label of the nucleotide or the analog thereof is ROX.

In some embodiments of the present disclosure, in step (ii) of the sequencing method, the four types of nucleotides or analogs thereof carrying the optically detectable labels are bound to the nucleic acid template-primer complex by using a polymerase to achieve the single-base extension, wherein the polymerase is a DNA polymerase and/or RNA polymerase, and the polymerase has a final concentration of 0.005-0.02 mg/mL.

In some embodiments of the present disclosure, the enzyme isselected from one or more of 9°N enzyme, Pfu, KOD1, and MMS2.

In some embodiments of the present disclosure, in step (ii) of the sequencing method, the polymerization reaction is performed at a pH of 8.0-9.0.

In some embodiments of the present disclosure, the optically detectable label is linked to the nucleotide or analog thereof via a chemically cleavable group, and in step (iv), removing the optically detectable label on the extension product is achieved by using a cleavage reagent including an organic phosphine and a buffer.

In some embodiments of the present disclosure, in step (iv) of the sequencing method, removing the optically detectable label on the extension product is performed at a pH value of 8.0-10.5 and a temperature of 39-65 °C.

In some embodiments of the present disclosure, the organic phosphine has a concentration of 10-100 mmol/L; and/or the buffer has a concentration of 0.01-2 mol/L.

In some embodiments of the present disclosure, the organic phosphine is selected from tris(3-hydroxypropyl)phosphine, tris(2-carboxyethyl)phosphine, tris(hydroxymethyl)phosphine, triphenylphosphine, and tris(2-hydroxyethyl)phosphine.

In some embodiments of the present disclosure, in step (iii), exciting the optically detectable label to generate the optical signal includes performing exposure processing on the solid carrier, wherein the exposure processing lasts for 100-400 ms.

In some embodiments of the present disclosure, the optically detectable labels comprise a first optically detectable label and a second optically detectable label, and excitation wavelengths of the first optically detectable label and the second optically detectable label differ by at least 50 nm. In some embodiments of the present disclosure, in the above sequencing method, the first optically detectable label is a red-light-excitable fluorophore, and the red-light-excitable fluorophore has an exposure time of 100-400 ms; the second optically detectable label is a green-light-excitable fluorophore, and the green-light-excitable fluorophore has an exposure time of 50-100 ms.

The sequencing method provided by the present disclosure has a lower sequencing error rate, is beneficial to improving the sequencing quality, and shortens the sequencing time.

### BRIEF DESCRIPTION OF THE DRAWINGS

In order to more clearly illustrate the technical schemes in the embodiments of the present application or the prior art, the drawings required for use in the description of the embodiments or the prior art will be briefly described below. It is obvious that the drawings in the description below are only some embodiments of the present application, and other drawings can be derived from these drawings by those of ordinary skill in the art without creative efforts.

Among the drawings:
FIG. 1 is a graph showing luminance results of dCTP-N3-IF700 and dGTP-N3-CY5 obtained by a 640 nm laser under different exposure times according to one embodiment of the present application;
FIG. 2 is a comparison graph showing a C fluorescent image obtained by one-color sequencing and a GC fluorescent image obtained by multi-color sequencing according to one embodiment of the present application;
FIG. 3 is a graph showing the effect of different exposure times on the distribution of 40-cycle read lengths according to one embodiment of the present application;
FIG. 4 is a graph showing the effect of different exposure times on the distribution of 50-cycle read lengths according to one embodiment of the present application;
FIG. 5 is a statistical diagram showing base cleavage efficiencies of four-color sequencing under THPP treatment at different concentrations according to one embodiment of the present application;
FIG. 6 is a statistical diagram showing base cleavage efficiencies of four-color sequencing with different concentrations of Tris buffer according to one embodiment of the present application;
FIG. 7 is a statistical diagram showing base cleavage efficiencies of four-color sequencing under different pH conditions according to one embodiment of the present application;
FIG. 8 is a statistical diagram showing base cleavage efficiencies of four-color sequencing at different temperatures according to one embodiment of the present application;
FIG. 9 is a graph showing the distribution of 50 cycles sequencing read lengths according to one embodiment of the present application;
FIG. 10 shows the distribution of error rates of 50 cycles of four-color sequencing according to one embodiment of the present application.

### DETAILED DESCRIPTION

The technical schemes in the embodiments of the present application will be described below clearly and comprehensively in conjunction with the drawings in the embodiments of the present application. It is obvious that the described embodiments are part of the embodiments of the present application, but not all of them. On the basis of the embodiments of the present application, all other embodiments obtained by those of ordinary skill in the art without creative efforts shall fall within the protection scope of the present application.

The present disclosure discloses a single-molecule sequencing method.

It should be understood that the expression "one or more of ..." individually includes each of the objects recited after the expression and various different combinations of two or more of the objects recited, unless otherwise understood from the context and usage. The expression "and/or" in combination with three or more of the recited objects should be understood to have the same meaning unless otherwise understood from the context.

The use of the terms "comprise", "include", "have", or "contain", including grammatical synonyms thereof, should generally be understood as open-ended and non-limiting, for example, without excluding other unrecited elements or steps, unless otherwise specifically stated or unless otherwise understood from the context.

It should be understood that the order of steps or the order for performing certain actions is not important so long as the present disclosure remains operable. In addition, two or more steps or actions may be performed simultaneously.

The use of any and all examples or exemplary language (e.g., "for example" or "including") herein is merely intended to better illustrate the present disclosure and is not intended to limit the scope of the present disclosure. The language in this specification should not be construed as indicating any unclaimed elements as essential for the practice of the present disclosure.

In addition, the numerical ranges and parameters defining the present disclosure are approximations, and the related numerical values in the specific embodiments have been presented as precisely as possible. Any numerical value, however, inherently contains a standard deviation necessarily resulting from certain methods of testing. Accordingly, unless otherwise explicitly indicated, it should be understood that all ranges, quantities, numerical values, and percentages used herein are modified by the word "about". Herein, "about" generally means that the actual numerical value is within a particular numerical value or range ± 10%, 5%, 1%, or 0.5%.

The terms "first", "second", "third", and "fourth" are used for descriptive purposes only, are used for distinguishing purposes such as positions, objects, and the like from one another, and should not be understood as indicating or implying relative importance or implicitly indicating the number of technical features indicated. For example, without departing from the scope of the embodiments in the present application, the terms "first" and "second" may be used interchangeably, for example, a first site may also be referred to as a second site, and correspondingly, a second site may be referred to as a first site. Therefore, features defined with "first", "second", "third", and "fourth" may explicitly or implicitly include one or more of the features.

The term "sequencing" may also be referred to as "nucleic acid sequencing" or "gene sequencing". The three are used interchangeably and refer to the determination of the type and order of bases or nucleotides (including nucleotide analogs) in a nucleic acid molecule. The sequencing involves the process of binding nucleotides to a template and acquiring the corresponding signals emitted by the nucleotides (including analogs). The sequencing includes sequencing by synthesis (SBS) and/or sequencing by ligation (SBL), including DNA sequencing and/or RNA sequencing.

The sequencing generally involves multiple cycles of process to achieve the determination of the type and order of multiple bases or nucleotides on a nucleic acid template. The embodiments of the present application refer to each cycle of the "process to achieve the determination of the type and order of multiple bases or nucleotides on a nucleic acid template" as "one cycle of sequencing". "One cycle of sequencing", also referred to as "sequencing cycle", may be defined as the completion of one base extension of four types of nucleotides/bases; in other words, "one cycle of sequencing" may be defined as the determination of the base or nucleotide type at any given position on the template. For sequencing platforms that achieve sequencing on the basis of polymerization or ligation reactions, one cycle of sequencing includes the process of binding four types of nucleotides (including nucleotide analogs) to the nucleic acid template at a time according to the base complementary rule and acquiring the corresponding signals emitted. For platforms that achieve sequencing on the basis of the polymerization reaction, a reaction system includes reaction substrate nucleotide, polymerase, and a nucleic acid template. A sequence fragment (a sequencing primer) binds to the nucleic acid template, and on the basis of the base pairing rules and the principle of polymerization reaction, the added reaction substrate nucleotides are linked to the sequencing primer under the catalysis of the polymerase to realize the binding of a nucleotide to a specific position on the nucleic acid template. Generally, one cycle of sequencing may include one or more base extensions (repeats). For example, four types of nucleotides are sequentially added to the reaction system to each perform base extensions and corresponding acquisition of reaction signals, and one cycle of sequencing includes four base extensions; for another example, four types of nucleotides are added into the reaction system in any combinations (such as in pairs or in one-three combinations), the two combinations each perform base extensions and corresponding acquisition of reaction signals, and one cycle of sequencing includes two base extensions; for yet another example, four types of nucleotides are added simultaneously to the reaction system for base extension and reaction signal acquisition, and one cycle of sequencing includes one base extension. A nucleotide refers to four types of natural nucleotides (e.g., dATP, dCTP, dGTP and dTTP, or ATP, CTP, GTP and UTP) or derivatives thereof, and is sometimes directly referred to as the base included (A, T/U, C, and G). The nucleotide or base represented by the expression will be known to those of ordinary skill in the art from the context.

The term "nucleic acid molecule" refers to polymeric forms of nucleotides of any length and may include ribonucleotides or analogs thereof, deoxyribonucleotides or analogs thereof, and mixtures of the above nucleotides or analogs thereof. The nucleic acid molecule may refer to a single-stranded polynucleotide or a double-stranded polynucleotide. Nucleotides in a nucleic acid molecule may include naturally occurring nucleotides and functionally alternative analogs thereof. Examples of analogs include those that can hybridize to nucleic acids in a sequence-specific manner, or can be used as templates for the replication of particular nucleotide sequences. Naturally occurring nucleotides generally have a backbone containing phosphodiester bonds. Analog structures may have alternative backbone linkages including any types known in the art. Naturally occurring nucleotides generally have deoxyribose (e.g., found in DNA) or ribose (e.g., found in RNA). Analog structures may have alternative sugar moieties including any types known in the art. Nucleotides may contain natural bases or non-natural bases. Bases in natural DNA may include one or more of adenine, thymine, cytosine, and/or guanine, and bases in natural RNA may include one or more of adenine, uracil, cytosine, and/or guanine. Any non-natural base or base analog may also be used for a nucleotide, such as a locked nucleic acid (LNA) and a bridged nucleic acid (BNA).

The term "nucleic acid template" refers to a master nucleic acid molecule or master nucleic acid molecule fragment that binds to nucleotides or nucleotide analogs by multiple successive base extension cycles. The nucleic acid template may be the entire sequence of the nucleic acid molecule or a partial fragment of the nucleic acid molecule. The "nucleic acid template" may be a nucleic acid fragment used as a template in extension reactions in which sequencing by synthesis is performed; since the base added to the extension reactions and the sequencing template satisfy the base pairing rules, the sequence of the sequencing template can be determined by determining the type of the base added to each extension cycle. The sequence of the nucleic acid template herein may generally include, but is not limited to, at least one of the sequence of the target molecule, the UMI sequence, and the sample tag sequence, sometimes also referred to herein as an "insert" or "target molecule".

The term "primer", also known as "probe", refers to an oligonucleotide or a nucleic acid molecule that can hybridize to a target sequence of interest. In an embodiment, the primer functions as a substrate onto which nucleotides may be polymerized by a polymerase. For example, the primer may be used as a starting point for DNA or RNA synthesis. For example, a sequencing primer can hybridize to the synthesized nucleic acid template strand so as to trigger the synthesis of a new strand complementary to the synthesized nucleic acid template strand. The primer may include any combination of nucleotides or analogs thereof. In some examples, the primer is a single-stranded oligonucleotide or polynucleotide.

In an embodiment of the present application, it should be understood that "XX or an analog thereof' includes "XX" and "XX analog". Illustratively, a nucleotide or an analog thereof includes a nucleotide and a nucleotide analog, a base or an analog thereof includes a base and a base analog, ribosyl or an analog thereof includes ribose and a ribose analog, and deoxyribosyl or an analog thereof includes deoxyribose and a deoxyribose analog.

The term "analog" or "functional analog" refers to a chemical compound that is structurally similar to another compound (i.e., a so-called "reference" compound) but has a different composition, for example, one atom is replaced by an atom of a different element, or a particular functional group is present differently, or one functional group is replaced by another functional group.

The term "FOV" refers to the field of view. The application of the solid substrate includes a process of performing multiple cycles of optical imaging (photographing) on a fixed region of the solid substrate by using an optical camera, and the region photographed by the optical camera each time is referred to as a FOV (field of view).

Taking the sequencing by synthesis as an example, the single-molecule sequencing method is described below. The single-molecule sequencing method provided in the embodiments of the present application includes:
(i) providing a solid carrier comprising a plurality of sites, each of the sites having a nucleic acid template-primer complex immobilized thereon.

In this step, the solid carrier is understood to be a carrier or support on which a nucleic acid molecule or nucleic acid template is immobilized. In some application examples, the solid carrier may also be referred to as a solid substrate, a sequencing chip, or a sequencing biochip. In one embodiment, the solid carrier is a substrate with an array of micropores, and each micropore is located at a position corresponding to a site. Illustratively, the solid carrier is a nanowell pattern chip, and the nanowell pattern chip includes nanowells arranged in an array for immobilizing the nucleic acid template-primer complex, wherein the nanowell may also be referred to as a nanopore, a micropore, or a pore. In an embodiment of the present application, the indicated nanowell and nanopore may be interchangeable, and refer to a nanoscale pore structure formed on the surface of the solid carrier. The shape of the nanowell or nanopore is not strictly limited, and the nanowell or nanopore may have a shape of a circle, an ellipse, a triangle, a quadrangle or other polygons, or even has no specific shape. In some embodiments, the nanowell or nanopore is configured to be a shape with a symmetrical cross-section. Illustratively, the cross-section of the nanowell or nanopore is a circle or regular polygon.

In one embodiment, the solid carrier is a planar substrate, and the surface of the planar substrate comprises several surface-processed positions, each position corresponding to a site for immobilizing the nucleic acid template-primer complex. Surface processing refers to forming an active functional group, which can be linked to the nucleic acid template-primer complex, on the surface. An example of this type of solid carrier is a random chip.

The solid carrier has a plurality of sites, and there is a certain distance between two adjacent sites. The distance is large enough so that optical signals generated by optically detectable labels at the adjacent sites can be distinguished.

In this embodiment, a nucleic acid template is immobilized on the surface of the solid carrier, and the nucleic acid template is linked to the surface of the solid carrier. In one embodiment, a probe is linked to the surface of the solid carrier, the probe is linked to a molecule or a group on the surface of the solid carrier via a covalent bond, and the nucleic acid template is linked to the surface of the solid carrier via the probe. The probe is an oligonucleotide or nucleic acid molecule fragment that can hybridize to a target nucleic acid molecule of interesting. In one embodiment, at least a portion of the probe is configured to hybridize to at least a portion of the 3' end of the nucleic acid template. In another embodiment, one end of the nucleic acid template is linked to a molecule or a group on the surface of the solid carrier via a covalent bond, thereby linking to the surface of the solid carrier. In an embodiment of the present application, the probe and the nucleic acid template jointly form the nucleic acid template-primer complex.

In this embodiment, the solid carrier has a plurality of sites, and correspondingly, the nucleic acid template is immobilized on the site of the solid carrier. According to the embodiments of the present application, ideally, one type of nucleic acid template is immobilized at each site. In some inevitable cases, there may be cases where very few sites have no nucleic acid template immobilized or where two types of different nucleic acid template are immobilized at one site. When the same site contains two types of different nucleic acid templates, the site produces two detectable signals in one cycle of sequencing. To improve the accuracy of sequencing, the sequencing signals at these sites are not counted, and the corresponding sequencing data are discarded.

In some embodiments, the solid carrier comprises runner that can communicate one end of the solid carrier to the other end, and in this case, the solution and/or reagent for the polymerization reaction in step (ii) and other solutions subsequently contacted with the solid carrier may be contacted by flowing through the surface of the runner. In one embodiment, the runner on the surface of the solid carrier may be formed on the surface of the solid carrier by, for example, etching. Materials are removed from a part of the surface of the solid carrier by, for example, etching, so that the part of the surface forms a groove structure. The groove structure leads from one end of the solid carrier to the other end of the solid carrier. The runner on the surface of the solid carrier can also be formed by covering a mask material layer on the surface of a solid substrate with a certain thickness, and the pattern of the mask material layer corresponds to the expected runner arranged on the surface of the solid carrier. For example, the mask material layer masks a region where the corresponding groove is located, hollow processing is performed on the other regions except the groove region, and then the material is deposited on the hollowed region of the mask material layer by using, for example, a chemical deposition method, so as to form a material layer with the runner on the surface of the solid carrier, and the solid carrier and the material layer formed on the surface of the solid carrier jointly constitute the solid carrier. It should be understood that the surface of the solid carrier may have one runner, or two or more, such as 4, 8, or 16, runnersw. Of course, the number of runner is not limited thereto. In some embodiments, the runner on the surface of the solid carrier is also referred to as Lane.

Illustratively, the runner has a width of 5-15 mm and a height of 50-200 µm. It should be understood that the width and height of the solid carrier may be arbitrarily combined within the ranges described above without strict limitations. Illustratively, when the width of the solid carrier is 5-10 mm, the height of the solid carrier may be in the range of 50-100 µm, 80-120 µm, 100-150 µm, 120-180 µm, 180-200 µm, or the like; of course, when the width of the solid carrier is 10-15 mm, the height of the solid carrier may be in the range of 50-100 µm, 80-120 µm, 100-150 µm, 120-180 µm, 180-200 µm, or the like.

(ii) Binding four types of nucleotides or analogs thereof carrying different optically detectable labels to the nucleic acid template-primer complex by a polymerization reaction to achieve single-base extension and obtain an extension product.

In this step, the nucleotides or the analogs thereof carrying different optically detectable labels are added to the surface of the solid carrier under conditions suitable for the polymerization reaction, so that the nucleotides or the analogs thereof pair with the nucleic acid template by the base complementary pairing rules and bind to the nucleic acid template-primer complex.

In some embodiments, each nucleotide or analog thereof has a final concentration of 125-1000 nmol/L. It should be understood that the final concentration of the nucleotide or the analog thereof indicated in the embodiments of the present application refers to the concentration of the nucleotide or the analog thereof when a solution containing the nucleotide or the analog thereof is contacted with the nucleic acid template-primer complex. Illustratively, the reaction solution containing the nucleotide or the analog thereof is passed through the solid carrier, and the concentration of the nucleotide or the analog thereof in the reaction solution is the final concentration of the nucleotide or the analog thereof. In one embodiment, this step involves pumping different reagents from different kits, mixing the reagents to form a mixed solution, and flowing the mixed solution onto the surface of the solid carrier, and in this case, the final concentration of the nucleotide or the analog thereof should be the concentration of the nucleotide or the analog thereof in the mixed solution. The final concentration of the nucleotide or the analog thereof is set to be 125-1000 nmol/L in the embodiments of the present application, so that the adsorption of the nucleotide or the analog thereof on other regions except the sites on the surface of the solid carrier is reduced, and the non-specific adsorption formed thereby is reduced under the polymerization reaction condition. The optically detectable labels in the nucleotides or the analogs thereof at these non-specific adsorption positions are excited in step (iii) to generate optical signals, which improves background noise in the images obtained by imaging, and interferes with the identification of the optical signals at the sites, further affecting the accuracy of base calling.

In the embodiments of the present application, the final concentration of each nucleotide or analog thereof may be adjusted in the range of 125-1000 nmol/L according to the type of the nucleotide or the analog thereof, the concentration of the polymerase in the reaction system, and the like.

In some embodiments, the nucleotide or the analog thereof may be represented by the general structure of formula 1 below:

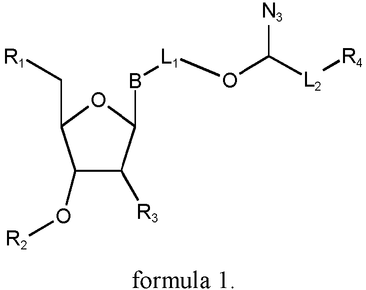

In formula 1, B is a base or an analog thereof. In some embodiments, B is cytosine or a derivative thereof, thymine or a derivative thereof, adenine or a derivative thereof, guanine or a derivative thereof, hypoxanthine or a derivative thereof, deazaadenine or a derivative thereof, deazaguanine or a derivative thereof, deazahypoxanthine or a derivative thereof, 7-methylguanine or a derivative thereof, 5,6-dihydrouracil or a derivative thereof, 5-methylcytosine or a derivative thereof, or 5-hydroxymethylcytosine or a derivative thereof.

According to the embodiments of the present disclosure, B is divalent cytosine or a derivative thereof, divalent guanine or a derivative thereof, divalent adenine or a derivative thereof, divalent thymine or a derivative thereof, divalent uracil or a derivative thereof, divalent hypoxanthine or a derivative thereof, divalent xanthine or a derivative thereof, divalent deazaadenine or a derivative thereof, divalent deazaguanine or a derivative thereof, divalent deazahypoxanthine or a derivative thereof, divalent 7-methylguanine or a derivative thereof, divalent 5,6-dihydrouracil or a derivative thereof, divalent 5-methylcytosine or a derivative thereof, or divalent 5-hydroxymethylcytosine or a derivative thereof.

In formula 1, L₁ is used to link B to a cleavable azide group, L₂ is used to link the cleavable azide group to R₄, and L₁ and L₂ are each independently a covalent bond or a covalent linker group. The cleavable azide group is bound to the base side of the nucleotide or the analog thereof, and generates steric hindrance on the 3'-hydroxyl site of ribose or deoxyribose by forming a certain spatial conformation, thereby blocking the polymerization reactivity of the site with other nucleotides or nucleotide analogs such as -O-CH(N₃)-L₂-R₄. The reversible terminating group can be removed (reduced into hydroxyl) by a chemical reaction under suitable conditions. Among them, the suitable conditions may be chemical reaction conditions that can initiate the reduction of the reversible terminating group into hydroxyl.

In some embodiments, L₁ is an independent bond, or substituted or unsubstituted C₁₋₈ alkylene, or substituted or unsubstituted 2- to 8-membered heteroalkylene, or substituted or unsubstituted C₃₋₈ cycloalkylene, or substituted or unsubstituted 3- to 8-membered isocycloalkylene, or substituted or unsubstituted C₆₋₈ arylene.

Illustratively, L₁ may be selected from the following structures: 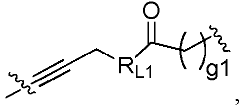 where g1 is an integer from 0 to 10, and R_{L1} is NH or O; g1 is preferably 1 or 4;
or 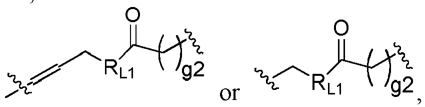 where g2 is 0, 1, 2, 3, 4, or 5, and R_{L1} is NH or O; g2 is preferably 1 or 4;
or 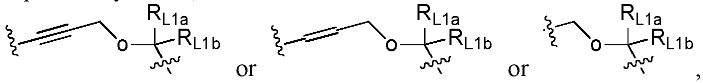 where R_{L1a} and R_{L1b} are each independently H, -CH₃, -CX₃, -CHX₂, -CH₂X, -CN, -Ph, C₁₋₆ alkyl, 2- to 6-membered alkyl, or 3- to 6-cycloalkyl, where X is Cl Br, or I;
or 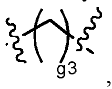 where g3 is 0, 1, 2, 3, or 4, preferably 1 or 2.

In some embodiments, L₂ is an independent bond, or substituted or unsubstituted alkyl, or substituted or unsubstituted heteroalkylene, or substituted or unsubstituted cycloalkyl, or substituted or unsubstituted isocycloalkyl, or substituted or unsubstituted aryl.

Illustratively, L₂ may include, but is not limited to, the following structures: 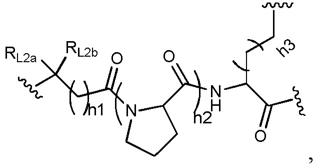 where R_{L2a} and R_{L2b} are each independently H, a C₁₋₅ alkyl chain, 3- to 6-membered cycloalkyl, or phenyl, preferably H, methyl, ethyl, 5- to 6-membered cycloalkyl, or phenyl; h1, h2, and h3 are each independently an integer from 0 to 6, preferably 0, 1, or 2;
or 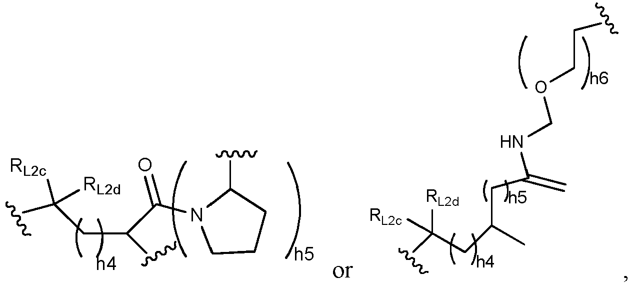 where R_{L2c} and R_{L2d} are each independently H or a C₁₋₆ alkyl chain; h4, h5, and h6 are each independently 0, 1, 2, 3, 4, 5, or 6; h4 is preferably 0, 1, or 2, h5 is preferably 5 or 6, and h6 is preferably 4, 5, or 6;
or 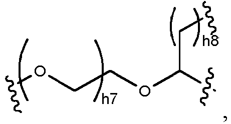 where h7 and h8 are each independently 1, 2, 3, 4, 5, or 6, preferably 1, 2, or 3.

In formula 1, R₁ is -OH or a phosphate group. In the embodiments of the present application, the "phosphate group" refers to a phosphoryl group, i.e., a group formed by removing one or two hydrogen atoms from monophosphoric acid or polyphosphoric acid, and has the following chemical structure, where n is an integer selected from 1 to 3:

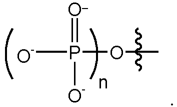

In formula 1, R₂ is H or a chemically cleavable group. When R₂ is a chemically cleavable group, it is bound to the 3'-hydroxyl site of ribose or deoxyribose and can inhibit the polymerization of the next nucleotide or analog thereof at this position, such that only one nucleotide or analog thereof can be introduced into a nucleic acid strand in each polymerization reaction of step (ii). Furthermore, the chemically cleavable group can be reversibly removed by a chemical reaction such as a cleavage reaction or the like under suitable conditions to restore the 3'-hydroxyl state of ribose or deoxyribose.

In some embodiments, R₂ is selected from one of H, -CH₂N₃, -CH(N₃)CH₂F, -CH(N₃)C(O)NHBu, -CH(N₃)CHF₂, -CH(N₃)C(O)OH, -CH(N₃)CH₂O(CH₂)₆NH₂, -CH(N₃)CH₂OArC(O)NH(CH₂)₆NH₂, and -C(R₁ₐR_{1b})-OC(R₂ₐR_{2b})R₇, where R₇ is selected from alkylene containing 1 to 8 carbon atoms. Illustratively, R₆ is selected from isomers of -(CH₂)ₛ- or -(CH₂)ₛ-, and s is selected from 1 to 8, specifically 1, 2, 3, 4, 5, 6, 7, or 8. Illustratively, R₇ may be -CH₂-, -(CH₂)₂-, -(CH₂)₃-, -(CH₂)₄-, - (CH₂)₅-, -(CH₂)₆-, or the like. When R₂ is selected from -C(R₁ₐR_{1b})-OC(R₂ₐR_{2b})R₇, R₁ₐ, and R_{1b} are each independently selected from H, a halogen atom, alkyl containing 1 to 6 carbon atoms, haloalkyl containing 1 to 6 carbon atoms, alkoxy containing 1 to 6 carbon atoms, haloalkoxy containing 1 to 6 carbon atoms, cyano, optionally substituted phenyl, or optionally substituted aralkyl, R₂ₐ and R_{2b} are each independently selected from H, a halogen atom, alkyl containing 1 to 6 carbon atoms, haloalkyl containing 1 to 6 carbon atoms, and cyano, or R₁ₐ, and R₂ₐ, together with the atoms to which they are linked, form optionally substituted five- to eight-membered alkylcyclyl or heterocyclyl; R₇ is selected from H, optionally substituted alkenyl containing 2 to 6 carbon atoms, optionally substituted cycloalkenyl containing 3 to 7 carbon atoms, optionally substituted alkynyl containing 2 to 6 carbon atoms, and optionally substituted silicon-containing groups.

In formula 1, R₃ is H or -OR₅, where R₅ is H or a chemically cleavable group. When R₅ is a chemically cleavable group, reference may be made to R₂ for the type.

In formula 1, R₄ contains the optically detectable label. The optically detectable label is a group that can generate a detectable optical signal under the excitation of light, where the optical signal can be a fluorescence signal. Illustratively, in one example, the detectable group is a fluorescent dye, and in this case, a nucleotide or an analog thereof containing the fluorescent dye can generate a detectable fluorescence signal under the action of excitation light. Illustratively, the optically detectable label includes one or more of Atto532, CY5, IF700, ROX, a rhodamine dye, a cyanine dye, a coumarin dye, or a phycoerythrin.

In the structure provided by formula 1 above, when the types of nucleotides or analogs thereof are different, the final concentrations participating in the polymerization reaction may be different. As a first implementation, B is selected from adenine or an analog thereof, and in this case, the nucleotide or the analog thereof is an adenine nucleotide or an analog thereof. In some embodiments, when the reaction system of the polymerization reaction in step (ii) contains an adenine nucleotide or an analog thereof, the adenine nucleotide or the analog thereof has a final concentration of 500-1000 nmol/L, specifically 125 nmol/L, 150 nmol/L, 175 nmol/L, 200 nmol/L, 225 nmol/L, 250 nmol/L, 275 nmol/L, 300 nmol/L, 325 nmol/L, 350 nmol/L, 375 nmol/L, 400 nmol/L, 425 nmol/L, 450 nmol/L, 475 nmol/L, 500 nmol/L, 525 nmol/L, 550 nmol/L, 575 nmol/L, 600 nmol/L, 625 nmol/L, 650 nmol/L, 675 nmol/L, 700 nmol/L, 725 nmol/L, 750 nmol/L, 775 nmol/L, 800 nmol/L, 825 nmol/L, 850 nmol/L, 875 nmol/L, 900 nmol/L, 925 nmol/L, 950 nmol/L, 975 nmol/L, 1000 nmol/L, or the like.

As a second implementation, B is selected from guanine or an analog thereof, and in this case, the nucleotide or the analog thereof is a guanine nucleotide or an analog thereof. In some embodiments, when the reaction system of the polymerization reaction in step (ii) contains a guanine nucleotide or an analog thereof, the guanine nucleotide or the analog thereof has a final concentration of 125-500 nmol/L, specifically 125 nmol/L, 150 nmol/L, 175 nmol/L, 200 nmol/L, 225 nmol/L, 250 nmol/L, 275 nmol/L, 300 nmol/L, 325 nmol/L, 350 nmol/L, 375 nmol/L, 400 nmol/L, 425 nmol/L, 450 nmol/L, 475 nmol/L, 500 nmol/L, or the like.

As a third implementation, B is selected from cytosine or an analog thereof, and in this case, the nucleotide or the analog thereof is a cytosine nucleotide or an analog thereof. In some embodiments, when the reaction system of the polymerization reaction in step (ii) contains a cytosine nucleotide or an analog thereof, the cytosine nucleotide or the analog thereof has a final concentration of 500-1000 nmol/L, specifically 125 nmol/L, 150 nmol/L, 175 nmol/L, 200 nmol/L, 225 nmol/L, 250 nmol/L, 275 nmol/L, 300 nmol/L, 325 nmol/L, 350 nmol/L, 375 nmol/L, 400 nmol/L, 425 nmol/L, 450 nmol/L, 475 nmol/L, 500 nmol/L, 525 nmol/L, 550 nmol/L, 575 nmol/L, 600 nmol/L, 625 nmol/L, 650 nmol/L, 675 nmol/L, 700 nmol/L, 725 nmol/L, 750 nmol/L, 775 nmol/L, 800 nmol/L, 825 nmol/L, 850 nmol/L, 875 nmol/L, 900 nmol/L, 925 nmol/L, 950 nmol/L, 975 nmol/L, 1000 nmol/L, or the like.

As a fourth implementation, B is selected from thymine or an analog thereof, and in this case, the nucleotide or the analog thereof is a thymine nucleotide or an analog thereof; or B is selected from a uracil or an analog thereof, and in this case, the nucleotide or the analog thereof is a uracil nucleotide or an analog thereof. In some embodiments, when the reaction system of the polymerization reaction in step (ii) contains thymine or an analog thereof, or when the reaction system of the polymerization reaction in step (ii) contains a uracil nucleotide or an analog thereof, the nucleotide or the analog thereof has a final concentration of 500-1000 nmol/L, specifically 125 nmol/L, 150 nmol/L, 175 nmol/L, 200 nmol/L, 225 nmol/L, 250 nmol/L, 275 nmol/L, 300 nmol/L, 325 nmol/L, 350 nmol/L, 375 nmol/L, 400 nmol/L, 425 nmol/L, 450 nmol/L, 475 nmol/L, 500 nmol/L, 525 nmol/L, 550 nmol/L, 575 nmol/L, 600 nmol/L, 625 nmol/L, 650 nmol/L, 675 nmol/L, 700 nmol/L, 725 nmol/L, 750 nmol/L, 775 nmol/L, 800 nmol/L, 825 nmol/L, 850 nmol/L, 875 nmol/L, 900 nmol/L, 925 nmol/L, 950 nmol/L, 975 nmol/L, 1000 nmol/L, or the like.

In the embodiments of the present application, four types of nucleotides or analogs thereof carrying different optically detectable labels are added simultaneously in step (ii) to bind to the nucleic acid template-primer complex. In this case, the optically detectable labels of the nucleotides or the analogs thereof simultaneously added are different, so that when the optical signal detection and imaging are performed in step (iii), the different nucleotides or analogs thereof bound to the nucleic acid template-primer complex can be distinguished and identified in the images or other data obtained by image-based processing.

In this step, the nucleotides or the analogs thereof are added by means of a solution, so that the nucleotides or the analogs thereof are uniformly reacted with the nucleic acid template-primer complex. In one implementation, a mixture solution of two types of nucleotides or analogs thereof carrying different optically detectable labels is contacted with the nucleic acid template-primer complex to enable the nucleotides or the analogs thereof to bind to the nucleic acid template-primer complex. In this case, the nucleotides or the analogs thereof, such as four types of nucleotides or analogs thereof of ATGC or AUGC, may combined in pairs, and one set of nucleotides or analogs thereof is added at each time to participate in the polymerization reaction, so that the set of nucleotides or analogs thereof binds to the nucleic acid template-primer complex to complete one single-base extension. The two types of nucleotides or analogs thereof are distinguished by two types of different optically detectable labels in the set of nucleotides or analogs thereof. Thereafter, another set of nucleotides or analogs thereof is added to participate in the polymerization reaction, so that the set of nucleotides or analogs thereof binds to the nucleic acid template-primer complex to complete the second single-base extension, and the two types of nucleotides or analogs thereof are distinguished by two types of different optically detectable labels in the set of nucleotides or analogs thereof. At this point, one cycle of sequencing is completed. In this case, the single-molecule sequencing is two-color sequencing.

In another implementation, a mixture solution of four types of nucleotides or analogs thereof carrying different optically detectable labels is contacted with the nucleic acid template-primer complex to enable the nucleotides or the analogs thereof to bind to the nucleic acid template-primer complex. In this case, the four types of nucleotides or analogs thereof such as ATGC or AUGC are simultaneously added to participate in the polymerization reaction, so that the four types of nucleotides or analogs thereof in the set of nucleotides or analogs thereof are each bound to the nucleic acid template-primer complex to complete one single-base extension. At this point, one cycle of sequencing is completed. In this case, the single-molecule sequencing is four-color sequencing.

It should be understood that three types of nucleotides or analogs thereof carrying different optically detectable labels may also be bound to the nucleic acid template-primer complex to achieve single-base extension; and the remaining one type of optically detectable labeled nucleotide or analog thereof is then bound to the nucleic acid template-primer complex to complete one cycle of sequencing.

It should be understood that the optically detectable labels carried by the different nucleotides or analogs thereof added simultaneously are different. In some embodiments, when the types of nucleotides or analogs thereof are different, the optically detectable labels carried are different. Illustratively, in the two-color sequencing and four-color sequencing, the nucleotide or the analog thereof is an adenine nucleotide or an analog thereof, and the optically detectable label of the nucleotide or the analog thereof is Atto532; the nucleotide or the analog thereof is a guanine nucleotide or an analog thereof, and the optically detectable label of the nucleotide or the analog thereof is CY5; the nucleotide or the analog thereof is a cytosine nucleotide or an analog thereof, and the optically detectable label of the nucleotide or the analog thereof is IF700; the nucleotide or the analog thereof is a thymine nucleotide or an analog thereof, and the optically detectable label of the nucleotide or the analog thereof is ROX. In this case, the optically detectable labels collocated with different nucleotides or analogs thereof can be more easily identified in step (iii), and especially when the single-molecule sequencing is four-color sequencing, the pairing of the nucleotides and the optically detectable labels is beneficial to improving the accuracy of base calling.

In step (ii) of the embodiment of the present application, "binding at least two types of nucleotides or analogs thereof carrying different optically detectable labels to the nucleic acid template-primer complex by the polymerization reaction to achieve the single-base extension and obtain the extension product" is performed in the presence of a polymerase for initiating the polymerization reaction of the nucleotides or the analogs thereof with the terminal nucleotides or nucleotide analogs of a complementary strand of the nucleic acid template. That is, the nucleotide or the analog thereof carrying the optically detectable label is bound to the nucleic acid template-primer complex by using the polymerase to achieve the single-base extension. The polymerase may be a DNA polymerase or an RNA polymerase, or may contain both a DNA polymerase and an RNA polymerase. In some embodiments, the polymerase is a DNA polymerase, including but not limited to, one or more of 9°N enzyme, Pfu, KOD 1, and MMS2.

In some embodiments, the polymerase has a final concentration of 0.005-0.02 mg/mL. The final concentration of the polymerase indicated in the embodiments of the present application refers to the concentration of the nucleotide or the analog thereof when a solution containing the polymerase is contacted with the solid carrier. Illustratively, the reaction solution containing the polymerase is passed through the solid carrier, and the concentration of the polymerase in the reaction solution is the final concentration of the nucleotide or the analog thereof. In one embodiment, this step involves pumping different reagents from different kits, mixing the reagents to form a mixed solution, and flowing the mixed solution onto the surface of the solid carrier, and in this case, the final concentration of the polymerase should be the concentration of the polymerase in the mixed solution. The final concentration of the polymerase is set to be 0.005-0.02 mg/mL in the embodiments of the present application, so that the adsorption of the nucleotide or the analog thereof on other regions except the sites on the surface of the solid carrier is reduced, and the non-specific adsorption formed thereby is reduced under the polymerization reaction condition. The optically detectable labels in the nucleotides or the analogs thereof at these non-specific adsorption positions are excited in step (iii) to generate optical signals, which improves background noise in the images obtained by imaging, and interferes with the identification of the optical signals at the sites, further affecting the accuracy of base calling.

Illustratively, the polymerase has a final concentration of 0.005 mg/mL, 0.006 mg/mL, 0.007 mg/mL, 0.008 mg/mL, 0.009 mg/mL, 0.01 mg/mL, 0.011 mg/mL, 0.012 mg/mL, 0.013 mg/mL, 0.014 mg/mL, 0.015 mg/mL, 0.016 mg/mL, 0.017 mg/mL, 0.018 mg/mL, 0.019 mg/mL, 0.02 mg/mL, or the like.

In some embodiments, the polymerization reaction in step (ii) is performed at a pH of 8.0-9.0. Therefore, the concentration of the nucleotide or the analog thereof, the concentration of the polymerase, and the pH condition of the reaction system participating in the polymerization reaction may be regulated and controlled, so that the adsorption of the nucleotide or the analog thereof on other regions except the sites on the surface of the solid carrier is effectively reduced, the non-specific adsorption formed thereby is reduced, and the background noise in the image obtained in step (iii) is reduced, thereby improving the accuracy of base calling.

In the embodiments of the present application, the reaction system of the polymerization reaction in step (ii) further comprises a solvent for dissolving the nucleotide or the analog thereof and the polymerase, and the solvent may be a buffer such as tris(hydroxymethyl)aminomethane buffer, glycine, ethanolamine, tetraethylethylenediamine, tetramethylethylenediamine, N-butyldiethanolamine, diethylaminoethanol, Hepes buffer, and N,N-dihydroxyethylglycine.

Step (ii) is performed by one single-base extension or two single-base extensions to achieve the binding of the four types of nucleotides or analogs thereof to the nucleic acid template-primer complex, and in this case, the solid carrier carries one type of optically detectable label at each site to which the nucleic acid template-primer is bound.

(iii) Exciting the optically detectable label to generate an optical signal, and imaging the extension product to obtain an image.

In this step, the optically detectable label on the surface of the solid carrier after the polymerization reaction is photoexcited to generate an optical signal such as a fluorescence signal. It should be understood that different optically detectable labels require different wavelengths of excitation light to generate the optical signal. In some embodiments, the optically detectable labels of the nucleotides or the analogs thereof added in step (ii) comprise at least a first optically detectable label and a second optically detectable label, and the excitation wavelengths of the first optically detectable label and the second optically detectable label differ by at least 50 nm to increase the difference in optical signals generated by the first optically detectable label and the second optically detectable label. The larger the difference in the excitation wavelengths of the first optically detectable label and the second optically detectable label, the more distinct the difference in the optical signals generated by the first optically detectable label and the second optically detectable label, and the easier the two optically detectable labels are to be identified.

In this step, exciting the optically detectable label to generate the optical signal includes performing exposure processing on the solid carrier. In some embodiments, the exposure processing lasts for 100-400 ms. The difference in the intensity of the optical signals generated by the optically detectable labels may be increased to a certain extent by controlling the exposure time, thereby facilitating the identification of the types of nucleotides or analogs thereof introduced at the sites and improving the accuracy of base calling. Illustratively, the exposure processing lasts for 100 ms, 120 ms, 150 ms, 180 ms, 200 ms, 220 ms, 250 ms, 280 ms, 300 ms, 320 ms, 350 ms, 380 ms, 400 ms, or the like. It should be understood that the time of the exposure processing may be adjusted according to the type of the optically detectable label, so as to achieve the purpose of increasing the difference in the intensity of the optical signals generated by the optically detectable labels. In one embodiment, the first optically detectable label is a red-light-excitable fluorophore, and the red-light-excitable fluorophore has an exposure time of 100-400 ms, such as 100 ms, 120 ms, 150 ms, 180 ms, 200 ms, 220 ms, 250 ms, 280 ms, 300 ms, 320 ms, 350 ms, 380 ms, and 400 ms; the second optically detectable label is a green-light-excitable fluorophore, and the green-light-excitable fluorophore has an exposure time of 50-100 ms, such as 50 ms, 60 ms, 70 ms, 80 ms, 90 ms, and 100 ms.

After the optically detectable label is excited to generate the optical signal, the extension product is imaged to obtain an image. The type of the nucleotide or the analog thereof participating in the polymerization reaction at each site is determined by the acquired image and the data obtained from the analysis of the optical signal based on the image, i.e., the base calling in the current cycle of the base extension reaction is achieved.

In some embodiments, the solid carrier may also be optically imaged prior to step (i) to determine the background noise present on the surface of the solid carrier.

(iv) Removing the optically detectable label on the extension product.

In this step, the optically detectable label on the extension product, specifically on the nucleotide or the analog thereof bound to the end of the extension product, is removed to avoid the optically detectable label on the nucleotide or the analog thereof bound to the end of the extension product interfering with the optical signal in the next step (iii) when the steps (i)-(iii) are repeated next time. In some embodiments, the optically detectable label is linked to the nucleotide or the analog thereof via a chemically cleavable group, and the optically detectable label on the extension product is removed by using a cleavage reagent. Illustratively, the chemically cleavable group on the extension product, specifically on the nucleotide or the analog thereof bound to the end of the extension product, is cut off by passing the cleavage reagent through the surface of the solid carrier, so that the optically detectable label linked via the chemically cleavable group is removed. Of course, it should be understood that other chemically cleavable groups on the nucleotide or the analog thereof bound to the end of the extension product may also be cleaved by the cleavage reagent.

As an implementation, the structural general formula of the nucleotide or the analog thereof is shown in formula 1, and in this case, when R₂ is a chemically cleavable group, it may be reduced to H by a cleavage reagent, i.e., the 3' of the pentose sugar is reduced to hydroxyl. Cleaving the chemically cleavable group by the cleavage reagent may restore the polymerization capability of the extension product, specifically of the nucleotide or the analog thereof bound to the end of the extension product.

In some embodiments, the cleavage reagent includes an organic phosphine and a buffer. As a reducing agent, the organic phosphine is used to reduce the chemically cleavable group, such that the chemically cleavable group is detached from the extension product to prepare for the next single-base extension. The preparation includes: in one aspect, the chemically cleavable group on the pentose sugar being reduced to hydroxyl, providing a reaction site for the binding of nucleotides or analogs thereof for the next polymerization reaction; in another aspect, an azidomethoxy group linked to L₁ being reduced, thereby cleaving -O-CH(N₃)-L₂-R₄ linked to the azidomethoxy group, and eliminating the inhibition of the polymerization reaction site on the pentose sugar by this group. The organic phosphine used for reducing the azidomethoxy group is selected from at least one of trivalent organic phosphines. In some embodiments, the organic phosphine is selected from at least one of tris(3-hydroxypropyl)phosphine, tris(2-carboxyethyl)phosphine, tris(hydroxymethyl) phosphine, triphenylphosphine, and tris(2-hydroxyethyl)phosphine. In some embodiments, the organic phosphine has a concentration of 10-100 mmol/L; specifically, the organic phosphine may have a concentration of 10 mmol/L, 15 mmol/L, 20 mmol/L, 25 mmol/L, 30 mmol/L, 35 mmol/L, 40 mmol/L, 45 mmol/L, 50 mmol/L, 55 mmol/L, 60 mmol/L, 65 mmol/L, 70 mmol/L, 75 mmol/L, 80 mmol/L, 85 mmol/L, 90 mmol/L, 95 mmol/L, 100 mmol/L, or the like.

In some embodiments, the buffer is selected from at least one of a primary amine buffer and a tertiary amine buffer. These buffers can serve as solvents for raw materials in the mixing reaction, and can provide a stable pH environment. Compared with the primary amine buffer, the tertiary amine buffer can provide a better and stable reaction environment for the reduction of the azidomethoxy group into hydroxyl. Illustratively, the buffer is selected from at least one of tris(hydroxymethyl)aminomethane buffer, glycine, ethanolamine, tetraethylethylenediamine, tetramethylethylenediamine, N-butyldiethanolamine, diethylaminoethanol, Hepes buffer, and N,N-dihydroxyethylglycine.

In some embodiments, the buffer has a concentration of 0.01-2 mol/L. The pH environment provided by the buffer at a concentration of 0.01-2 mol/L is beneficial to improving the uniformity and stability of the reduction reaction. Illustratively, the buffer in the mixed solution has a concentration of 10 mmol/L, 15 mmol/L, 20 mmol/L, 25 mmol/L, 30 mmol/L, 35 mmol/L, 40 mmol/L, 45 mmol/L, 50 mmol/L, 55 mmol/L, 60 mmol/L, 65 mmol/L, 70 mmol/L, 75 mmol/L, 80 mmol/L, 85 mmol/L, 90 mmol/L, 95 mmol/L, 100 mmol/L, 105 mmol/L, 110 mmol/L, 115 mmol/L, 120 mmol/L, 125 mmol/L, 130 mmol/L, 135 mmol/L, 140 mmol/L, 145 mmol/L, 150 mmol/L, 155 mmol/L, 160 mmol/L, 165 mmol/L, 170 mmol/L, 175 mmol/L, 180 mmol/L, 0.2 mol/L, 0.3 mol/L, 0.4 mol/L, 0.5 mol/L, 0.6 mol/L, 0.7 mol/L, 0.8 mol/L, 0.9 mol/L, 1.0 mol/L, 1.1 mol/L, 1.2 mol/L, 1.3 mol/L, 1.4 mol/L, 1.5 mol/L, 1.6 mol/L, 1.7 mol/L, 1.8 mol/L, 1.9 mol/L, 2.0 mol/L, or the like.

In some embodiments, removing the optically detectable label on the extension product is performed at a pH value of 8.0-10.5 and a temperature of 39-65 °C. When the pH value is 8.0-10.5 and the reaction temperature is 39-65 °C, the structure of the nucleic acid strand is stable, and this temperature can also provide good reduction efficiency for the chemically cleavable group. Illustratively, the reaction temperature of the cleavage reagent may be 39 °C, 40 °C, 42 °C, 45 °C, 48 °C, 50 °C, 53 °C, 55 °C, 57 °C, 60 °C, 62 °C, 65 °C, or the like. Illustratively, the pH value of the cleavage reagent is 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.1, 10.2, 10.3, 10.4, 10.5, or the like.

After step (iv), the embodiments of the present application further include: performing cleaning treatment by using a cleaning reagent to remove the cleavage reagent and avoid organic phosphine residues. If the organic phosphine remains in steps (i)-(iii) of the current single-base extension, the organic phosphine may reduce the chemically cleavable group of the nucleotide or the analog thereof and -O-CH(N₃)- in the next single-base extension, resulting in that the single extension reaction is not terminated, and thus the number of nucleotides or analogs thereof incorporated into the nucleic acid template-primer complex cannot be effectively controlled, generating prephasing (or referred to as prephase, which means that, when sequencing, nucleotides or analogs thereof introduced in the next base extension or next consecutive base extensions undergo the polymerization reaction in advance during the current base extension and bind to the complementary strand of the nucleic acid template), and not accurately identifying the nucleic acid sequence of the introduced nucleotide region; or in the process of a new base extension reaction, the extension product after the nucleotide or the analog thereof is introduced has not been imaged, and the organic phosphine has already reduced the chemically cleavage group, so that the optically detectable label is detached, and the type of the nucleotide or the analog thereof introduced in this single-base extension reaction cannot be identified.

In another aspect, there is a selection principle of linker groups for the side chain and 3'-OH. The group used in the embodiments is azide, which is a cleavable group, however, in practical applications, without being limited to this group, other cleavable blocking groups may be tried and applied, several of which are shown in the patent EP3388442A1: -CH₂F, -C(O)NHBu, -CHF₂, - C(O)OH, -CHF₂, -C₆, and -ArC₆. All the groups may be cleavable by the reducing agent tris(hydroxymethyl)phosphine. Furthermore, acetal may also be used as a blocking group, but the cleavage of this group requires the use of a palladium catalyst, and the cleavage reagents provided in this patent are not effective in cleaving the acetal group. The groups linked to the side chain and 3'-OH may be selected from the same group as shown in the patent, or may be selected from different groups. However, in the case of selecting different groups, the selected groups are required to be effectively cleaved by the same reagent at the same time, and the 3' end is in a hydroxyl state after the blocking group is cleaved.

Taking four-color sequencing as an example, the present disclosure is further illustrated below. The four types of nucleotides used for sequencing in the embodiments of the present disclosure are represented as: dATP-N3-Atto532, dTTP-N3-ROX, dCTP-N3-IF700, and dGTP-N3-CY5, respectively, the structural general formula of which is shown as follows: 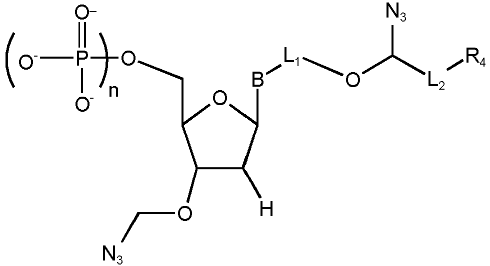 where B is A, T, G, or C, L₁ and L₂ are each independently a covalent bond or a covalent linker group, R₄ contains Atto532, ROX, IF700, or CY5, and n is an integer selected from 1 to 3. The excitation and emission wavelengths of Atto532, ROX, IF700, or CY5 are shown in Table 1 below.

**Table 1**

| Optically detectable label | Excitation wavelength (nm) | Emission wavelength (nm) |
|---|---|---|
| Atto532 | 532 | 552 |
| ROX | 578 | 604 |
| IF700 | 690 | 713 |
| CY5 | 651 | 670 |

### Example 1. Adsorption Assay for Single-Molecule Four-Color Sequencing

On a single-molecule sequencing platform, the non-specific adsorption of nucleotides or analogs thereof on the surface of a solid carrier has great interference on the identification of base signals during sequencing, affecting the sequencing quality. Thus, for the nucleotides or the analogs thereof used for sequencing, the appropriate concentrations of the four types of nucleotides and polymerases during the base extension reaction may be determined by the adsorption assay.

The method for the adsorption assay was as follows:
providing a surface-processed solid carrier comprising a plurality of sites, each of the sites having the ability to immobilize a nucleic acid template-primer complex, but not immobilizing the nucleic acid template-primer complex;
preparing nucleotide solutions of dATP-N3-Atto532, dTTP-N3-ROX, dCTP-N3-IF700, and dGTP-N3-CY5, respectively, adding 9°N enzyme to each nucleotide solution to form extension reagents, designating the extension reagent containing dATP-N3-Atto532 and 9°N enzyme as dATP-N3-Atto532 extension reagent, designating the extension reagent containing dTTP-N3-ROX and 9°N enzyme as dTTP-N3-ROX extension reagent, designating the extension reagent containing dCTP-N3-IF700 and 9°N enzyme as dCTP-N3-IF700 extension reagent, and designating the extension reagent containing dGTP-N3-CY5 and 9°N enzyme as dGTP-N3-CY5 extension reagent; setting different nucleotide concentrations and 9°N enzyme concentrations in the extension reagents;
processing the surface of the solid carrier by adopting excitation light with the same excitation wavelength as that of a fluorophore carried by the extension reagent to be added, and imaging the solid carrier after the excitation processing to obtain a first image; separately adding the extension reagents to the surface of the solid carrier, exciting the fluorophores on the extension reagents to generate optical signals, and imaging the solid carrier to obtain images; and
performing data analysis on the images, and counting the average number of fluorescence signal points (number of adsorption points) in the images. The results are shown in Table 2 below.

**Table 2**

| Type and concentration of nucleotide | Concentration of 9°N enzyme (mg/mL) | Number of adsorption points |
|---|---|---|
| dATP-N3-Atto532 500 nmol/L | 0 | 132.9 |
| dATP-N3-Atto532 500 nmol/L | 0.01 | 245.7 |
| dATP-N3-Atto532 750 nmol/L | 0.01 | 340.5 |
| dATP-N3-Atto532 1000 nmol/L | 0.01 | 484.5 |
| dTTP-N3-ROX 500 nmol/L | 0 | 291.6 |
| dTTP-N3-ROX 500 nmol/L | 0.01 | 340.9 |
| dTTP-N3-ROX 750 nmol/L | 0.01 | 402.3 |
| dTTP-N3-ROX 1000 nmol/L | 0.01 | 455.0 |
| dCTP-N3-IF700 500 nmol/L | 0 | 216.6 |
| dCTP-N3-IF700 500 nmol/L | 0.01 | 219.8 |
| dCTP-N3-IF700 750 nmol/L | 0.01 | 290.8 |
| dCTP-N3-IF700 1000 nmol/L | 0.01 | 391.8 |
| dGTP-N3-CY5 500 nmol/L | 0 | 217.4 |
| dGTP-N3-CY5 500 nmol/L | 0.01 | 478.3 |
| dGTP-N3-CY5 250 nmol/L | 0.01 | 406.6 |
| dGTP-N3-CY5 125 nmol/L | 0.01 | 387.3 |
| dGTP-N3-CY5 75 nmol/L | 0.01 | 254.9 |

As shown in Table 2, when the concentrations of dATP-N3-Atto532, dTTP-N3-ROX, and dCTP-N3-IF700 were 500-1000 nmol/L, the concentration of the dGTP-N3-CY5 nucleotide solution was 125-500 nmol/L, and the concentration of 9°N was 0.01 mg/mL, the number of adsorption points was less than 500. The adsorption ratio of the solid carrier surface was calculated according to (the number of adsorption points/the total number of sites on the carrier surface) ×100%, and an adsorption ratio within 2% was beneficial to improving the accuracy of base calling, finally improving the sequencing quality.

### Example 2. Single-Molecule Four-Color Sequencing

The method for the single-molecule four-color sequencing was as follows:
providing a surface-processed solid carrier comprising a plurality of sites, each of the sites having an immobilized nucleic acid template-primer complex;
preparing nucleotide solutions of dATP-N3-Atto532 at a concentration of 500 nmol/L, dTTP-N3-ROX at a concentration of 500 nmol/L, dCTP-N3-IF700 at a concentration of 1000 nmol/L, and dGTP-N3-CY5 at a concentration of 125 nmol/L, respectively, and adding 9°N enzyme at a concentration of 0.01 mg/mL to form extension reagents; designating the extension reagent containing dTTP-N3-ROX and 9°N enzyme as dTTP-N3-ROX extension reagent, designating the extension reagent containing dCTP-N3-IF700 and 9°N enzyme as dCTP-N3-IF700 extension reagent, and designating the extension reagent containing dGTP-N3-CY5 and 9°N enzyme as dGTP-N3-CY5 extension reagent; setting different nucleotide concentrations and 9°N enzyme concentrations in the extension reagents; and
adding the extension reagents to the surface of the solid carrier, separately adopting excitation light from a 532 nm laser and a 640 nm laser to irradiate the surface of the solid carrier and excite fluorophores on the extension reagents to generate optical signals, imaging the solid carrier, and acquiring images.

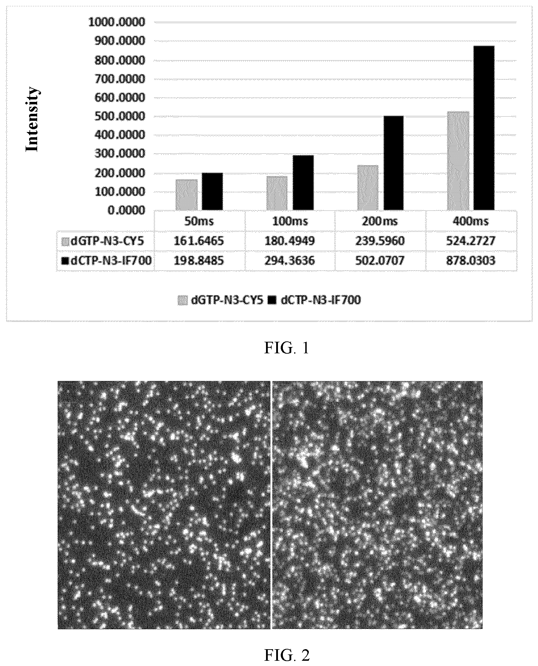

Given the image definition and limitations of the single molecule itself, multi-color sequencing is algorithmically more difficult to distinguish different types of nucleotide images, especially GC images. In this example, four exposure times of 50 ms, 100 ms, 200 ms, and 400 ms were set for the 640 nm laser, and the difference in luminance between dCTP-N3-IF700 and dGTP-N3-CY5 was observed. The results are shown in FIG. 1. As can be seen, when the exposure time was 50 ms, the fluorescence intensities of dCTP-N3-IF700 and dGTP-N3-CY5 were close; as the exposure time increased, the difference in fluorescence intensity between dCTP-N3-IF700 and dGTP-N3-CY5 increased. For the 640 nm laser, the exposure time of 100 ms or more may better distinguish GC on the image. The difference in fluorescence intensity of multiple types of nucleotides such as GC in the same fluorescence channel during the multi-color sequencing may be improved by increasing the exposure time. Specifically, as shown in FIG. 2, under an exposure time of 50 ms for the 640 nm laser, the fluorescent image formed by the reaction of dCTP-N3-IF700 alone (in one-color sequencing) is shown in the left panel of FIG. 2, and the GC fluorescent image acquired after adding the four types of nucleotides is shown in the right panel of FIG. 2. As can be seen, after G was subjected to crosstalk to the C image, it was more difficult to distinguish GC points by luminance in the C image. In this example, the images difficult to algorithmically distinguish were distinguished by adjusting the exposure time, e.g., adjusting the exposure time of the red-light-excitable fluorophore to be 100-400 ms and the exposure time of the green-light-excitable fluorophore to be 50-100 ms.

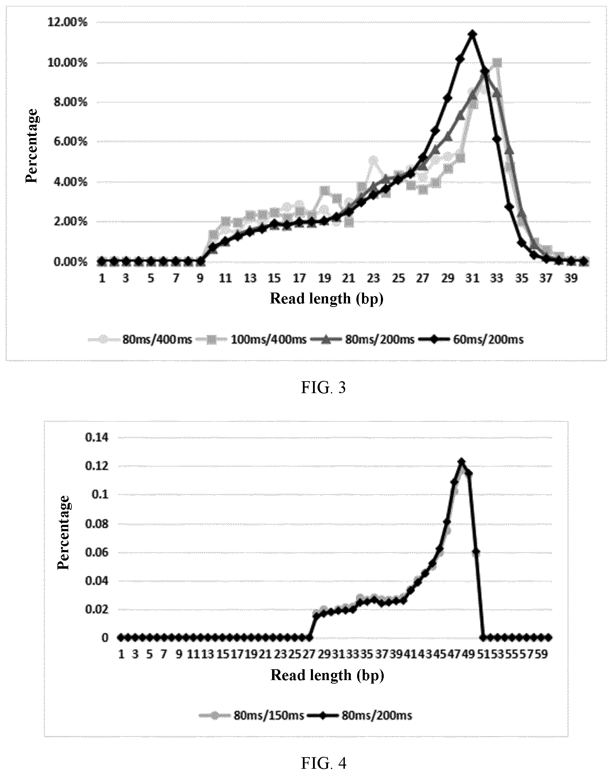

40-Cycle base extension was performed by adopting 532 nm and 640 nm lasers to verify different exposure times, and the effects of four different exposure conditions (exposure at 532 nm for 80 ms and exposure at 640 nm for 400 ms, exposure at 532 nm for 100 ms and exposure at 640 nm for 400 ms, exposure at 532 nm for 80 ms and exposure at 640 nm for 200 ms, and exposure at 532 nm for 60 ms and exposure at 640 nm for 200 ms) on the distribution of 40-cycle read lengths were analyzed. The results are shown in FIG. 3. As can be seen from FIG. 3, the exposure time had an effect on the sequencing read length. Specifically, as the exposure time increased, the photo damage increased, resulting in a shorter read length. Therefore, a shorter exposure time was selected on an algorithmically distinguishable basis.

The exposure time was set to 80 ms when using the 532 laser, the 640 nm laser was used for the exposure for 150 ms and 200 ms, and the 50 cycles of base extension were performed. The sequencing data are shown in Table 3 below (the number of Reads uniquely aligned to the genome refers to the number of Reads uniquely aligned to the reference genome within a 1024 × 1024 pixel region in the center of 1 field of view, i.e., one FOV), where the error rate = mismatch rate + deletion-type error rate + insertion-type error rate. As can be seen, the sequencing data obtained in the 50 cycles of base extension for the exposure times of 150 ms and 200 ms had little difference. The distribution results of the 50-cycle read lengths under different exposure times are shown in FIG. 4, and it can be seen that there was no significant difference in the read length distribution between exposures for 150 ms and 200 ms. Combining with the long sequencing result, when the 532 laser was used for 80 ms and the 640 laser was used for 150 ms, dCTP-N3-IF700 and dGTP-N3-CY5 could be distinguished from luminance, and the effect on the read length was small. Eventually, the main peak of the 50-cycle sequencing read length distribution was about 50 bp, the error rate was 7%-8%, and the four-color sequencing could be achieved on the single-molecule sequencing platform. Therefore, based on the consideration of saving the sequencing time, the exposure time of 150 ms could be adopted for the subsequent 640 nm laser.

**Table 3**

| 640 nm laser | Number of Reads uniquely aligned to genome | Mismatch rate | Deletion-type error rate | Insertion-type error rate | Error rate | Density (reads/µm²) |
|---|---|---|---|---|---|---|
| 150ms | 6470.6 | 3.01% | 3.25% | 1.38% | 7.65% | 5.5081 |
| 200ms | 6666.8 | 3.17% | 3.10% | 1.33% | 7.60% | 5.50759 |

### Example 3. Single-Molecule Four-Color Sequencing

The method for the single-molecule four-color sequencing was as follows:
providing a surface-processed solid carrier comprising a plurality of sites, each of the sites having an immobilized nucleic acid template-primer complex;
preparing nucleotide solutions of dATP-N3-Atto532 at a concentration of 500 nmol/L, dTTP-N3-ROX at a concentration of 500 nmol/L, dCTP-N3-IF700 at a concentration of 1000 nmol/L, and dGTP-N3-CY5 at a concentration of 125 nmol/L, respectively, and adding 9°N enzyme at a concentration of 0.01 mg/mL to form extension reagents, designating the extension reagent containing dATP-N3-Atto532 and 9°N enzyme as dATP-N3-Atto532 extension reagent, designating the extension reagent containing dTTP-N3-ROX and 9°N enzyme as dTTP-N3-ROX extension reagent, designating the extension reagent containing dCTP-N3-IF700 and 9°N enzyme as dCTP-N3-IF700 extension reagent, and designating the extension reagent containing dGTP-N3-CY5 and 9°N enzyme as dGTP-N3-CY5 extension reagent; setting different nucleotide concentrations and 9°N enzyme concentrations in the extension reagents; and
adding the extension reagents to the surface of the solid carrier, separately adopting excitation light from a 532 laser and a 640 laser to irradiate the surface of the solid carrier and excite fluorophores on the extension reagents to generate optical signals, imaging the solid carrier, and acquiring images.

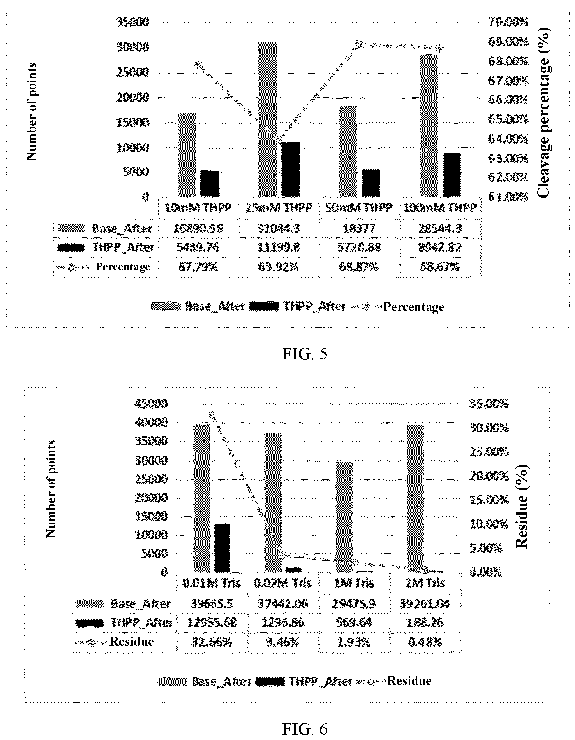

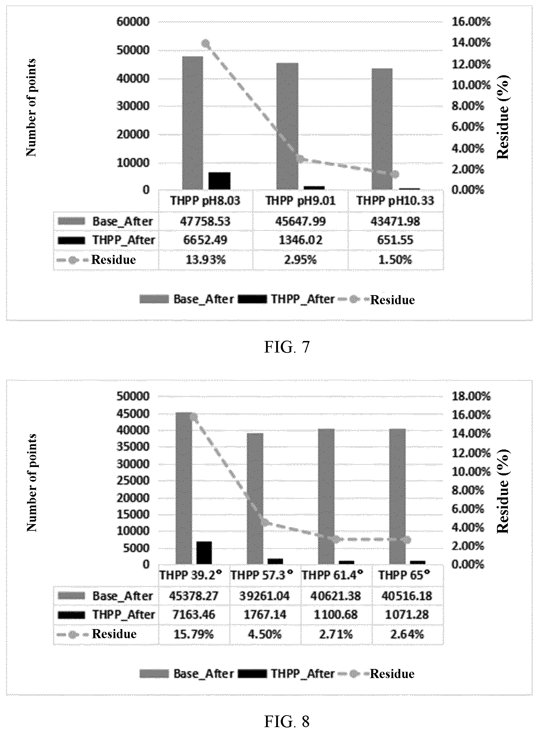

A Tris buffer system containing THPP was prepared as a cleavage reagent, the cleavage reagent was added to the surface of the solid carrier, and a cleavage reaction was performed on azidomethoxy. The cleavage percentage of the azidomethoxy on the surface of the solid carrier was counted (the number of optical signal points before and after cleavage was counted; the cleavage percentage of the azidomethoxy = (the number of optical signal points before cleavage - the number of optical signal points after cleavage)/the number of optical signal points before cleavage × 100%), or the residual percentage of the azidomethoxy on the surface of the solid carrier was counted (the number of optical signal points before and after cleavage was counted; the residual percentage of the azidomethoxy = the number of optical signal points after cleavage/the number of optical signal points before cleavage × 100%).
(1) In the cleavage reagents, the concentration of Tris was 2.0 mol/L, and four groups of THPP concentrations were set, which were 10 mmol/L, 25 mmol/L, 50 mmol/L, and 100 mmol/L, respectively; and the optical signals before and after the cleavage reaction under the four concentration conditions were counted, as shown in FIG. 5, and calculated that the cleavage percentages under the four concentration conditions were 67.79%, 63.92%, 68.87%, and 68.67%, respectively. It can be seen that the improvement of the THPP concentration had no significant effect on the improvement of the cleavage efficiency.
(2) In the cleavage reagents, the concentration of THPP was 10.0 mmol/L, and four groups of Tris buffer concentrations were set, which were 0.01 mol/L, 0.2 mol/L, 1 mol/L, and 2 mol/L, respectively; and the optical signals before and after the cleavage reaction under the four concentration conditions were counted, as shown in FIG. 6. FIG. 6 shows that the residual percentages after the cleavage reaction under the four concentration conditions were 32.66%, 3.46%, 1.93%, and 0.48%, respectively. It can be seen that the concentration of the Tris buffer affected the removal of the azidomethoxy, thereby affecting the sequencing error rate. When the concentration of the Tris buffer was 0.2-2.0 mol/L, the azidomethoxy had a lower residual percentage.
(3) In the cleavage reagents, under a condition that the concentration of Tris was 2.0 mol/L and the concentration of THPP was 10.0 mmol/L, pH was set to 8.0, 9.0, and 10.3, and the optical signals before and after the cleavage reaction under the three pH conditions were counted, as shown in FIG. 7. FIG. 7 shows that the residual percentages after the cleavage reaction under the three pH conditions were 13.93%, 2.95%, and 1.50%, respectively. It can be seen that the pH affected the removal of the azidomethoxy, thereby affecting the sequencing error rate. When the pH was 9 or higher, the azidomethoxy had a lower residual percentage. Considering that the stability of the nucleic acid molecule was affected by too high a pH, the pH value was preferably 9-10.3 during the four-color sequencing.
(4) In the step of performing the cleavage reaction on the azidomethoxy, a cleavage reagent containing Tris at a concentration of 2.0 mol/L and THPP at a concentration of 10.0 mmol/L with a pH value of 9.0 was used; the cleavage reaction was performed under temperature conditions of 39 °C, 57 °C, 61 °C, and 65 °C; and the optical signals before and after the cleavage reaction under the four temperature conditions were counted, as shown in FIG. 8. FIG. 8 shows that the residual percentages after the cleavage reaction under the four temperature conditions were 15.79%, 4.50%, 2.71%, and 2.64%, respectively. It can be seen that the temperature affected the removal of the azidomethoxy, thereby affecting the sequencing error rate. When the temperature was 39 °C or higher, the azidomethoxy had a lower residual percentage. Considering that the stability of the nucleic acid molecule was affected by too high a temperature, the temperature was preferably 39-65 °C during the four-color sequencing.

The cleavage reagent was optimized through the THPP concentration gradient, Tris concentration gradient, pH gradient, and temperature gradient. Among them, the increase in Tris concentration could significantly reduce the residues after cleavage. Under the condition of Tris at a high concentration of 2 mol/L, the increase in pH and temperature was also beneficial to further reducing the residues after cleavage, and finally, the cleavage efficiency of THPP was 95% or more.

### Example 4. Single-Molecule Four-Color Sequencing

The method included:
(1) providing a surface-processed solid carrier comprising a plurality of sites, each of the sites having an immobilized nucleic acid template-primer complex;
(2) preparing nucleotide solutions of dATP-N3-Atto532 at a concentration of 500 nmol/L, dTTP-N3-ROX at a concentration of 500 nmol/L, dCTP-N3-IF700 at a concentration of 1000 nmol/L, and dGTP-N3-CY5 at a concentration of 125 nmol/L, respectively (buffers: 50 mmol/L Tris-Base, pH 8.8; 50 mmol/L NaCl; 10 mmol/L (NH₄)₂SO₄; and 3 mmol/L MgSO₄), and adding 9°N enzyme at a concentration of 0.01 mg/mL to form extension reagents;
(3) adding the extension reagents to the surface of the solid carrier, adopting excitation light from a 532 laser and a 640 laser to irradiate the surface of the solid carrier for 80 ms and 150 ms, respectively, imaging the solid carrier, and acquiring images; and
(4) preparing a cleavage reagent containing the following components: 2 mol/L Tris-Base, 1 mol/L sodium chloride, 10 mmol/L THPP, 0.05% Tween 20, with a pH of 9.0, adding the cleavage reagent to the surface of the solid carrier, and performing a cleavage reaction on azidomethoxy.

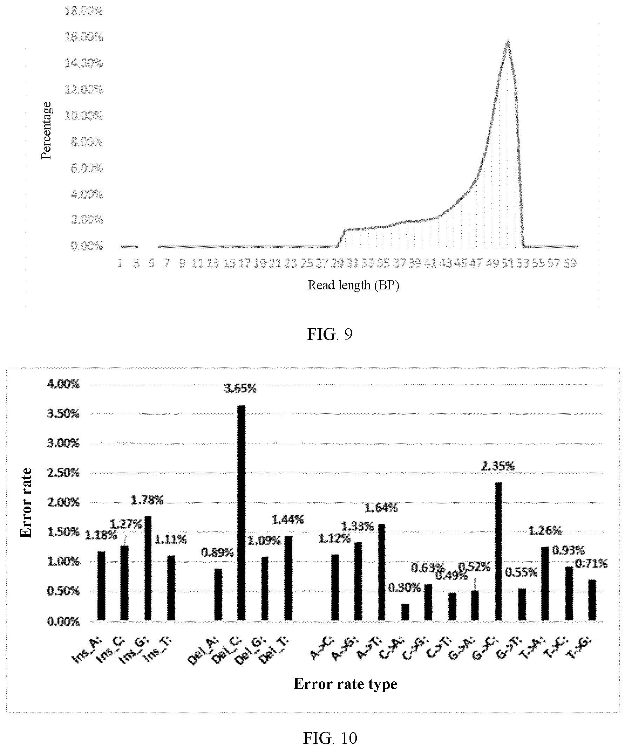

Steps (1)-(4) were repeated 50 times, i.e., 50 cycles of sequencing, and the distribution of 50-cycle read lengths is shown in FIG. 9. It can be seen from the figure, the main peak of the 50-cycle sequencing read length distribution was about 50 bp, and the proportion of sequencing terminated before 45 cycles was relatively small.

The error rates during the 50 cycles were counted, and the results are shown in Table 4 below, where the number of Reads uniquely aligned to the genome referred to the number of Reads uniquely aligned to the reference genome within a 1024 × 1024 pixel region in the center of 1 field of view, i.e., one FOV, and the error rates (error rate = mismatch rate + deletion-type error rate + insertion-type error rate) were about 6%.

**Table 4**

| Condition | Number of Reads uniquely aligned to genome (1024 × 1024 pixel region in the center of 1 field of view) | Mismatch rate | Deletion-type error rate | Insertion-type error rate | Error rate | Density (reads/µm²) |
|---|---|---|---|---|---|---|
| 125nM dG-CY5 | 7506.45 | 2.88% | 2.61% | 1.04% | 6.52% | 4.25 |
| 10nM dG-Atto647N | 8045.85 | 3.03% | 1.96% | 1.06% | 6.04% | 4.50 |
| 10nM dG-Atto647N | 7488.35 | 3.23% | 2.17% | 1.36% | 6.77% | 5.19 |
| 5nM dG-Atto647N | 7984.95 | 3.21% | 2.20% | 1.29% | 6.70% | 5.15 |

The statistical results of the four-color sequencing error types are shown in FIG. 10, where Ins_N indicates an error with an insertion type of N, Del_N indicates an error with a deletion type of N, N1- > N2 indicates that N1 is erroneously identified as an error type of N2, and N, N1, and N2 all indicate one of A, T, G, and C. As can be seen from FIG. 10, the error type and the distribution ratio thereof during the 50 cycles of four-color sequencing were within the acceptable range for the single-molecule sequencing.

The dG-CY5 base was replaced by the dG-Atto647N base, the reaction efficiency was high, and the error rate statistics are shown in Table 4. The error rates after dG-Atto647N replacement were approximate, but the throughput was slightly higher, and dG-Atto647N could be used as a substitute for dG-CY5.

In conclusion, the four-color sequencing could be achieved on the single-molecule platform, and better accuracy and a longer read length could be obtained.

The above description is only of the preferred embodiments of the present disclosure, and it should be noted that those of ordinary skill in the art can make, without departing from the principles of the present disclosure, various improvements and modifications, and such modifications and adaptations shall also be construed within the protection scope of the present disclosure.

## Claims

1. A single-molecule sequencing method, comprising the following steps:
(i) providing a solid carrier comprising a plurality of sites, each of the sites having a nucleic acid template-primer complex immobilized thereon;
(ii) binding four types of nucleotides or analogs thereof carrying different optically detectable labels to the nucleic acid template-primer complex by a polymerization reaction to achieve single-base extension and obtain an extension product;
(iii) exciting the optically detectable label to generate an optical signal, and imaging the extension product to obtain an image;
(iv) removing the optically detectable label on the extension product; and
(v) replacing the nucleic acid template-primer complex with the extension product from which the optically detectable label is cleaved, and repeating (ii) to (iv) above one or more times to determine a template nucleic acid sequence.

2. The sequencing method according to claim 1, wherein in step (ii), a mixture solution of the two or four types of nucleotides or analogs thereof carrying different optically detectable labels is contacted with the nucleic acid template-primer complex to enable the nucleotides or the analogs thereof to bind to the nucleic acid template-primer complex.

3. The sequencing method according to claim 2, wherein in step (ii), the four types of nucleotides or analogs thereof have a final concentration of 125-1000 nmol/L, respectively.

4. The sequencing method according to any one of claims 1 to 3, wherein the nucleotides or the analogs thereof have a general formula shown in formula 1: 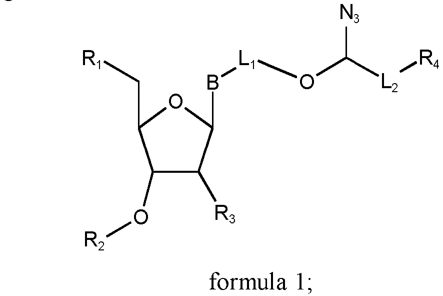 wherein B is a base or an analog thereof, L₁ and L₂ are each independently a covalent bond or a covalent linker group, R₁ is -OH or a phosphate group, R₂ is H or a chemically cleavable group, R₃ is H or -OR₅, R₅ is H or a chemically cleavable group, and R₄ comprises the optically detectable label.

5. The sequencing method according to claim 4, wherein B is selected from adenine or an analog thereof, guanine or an analog thereof, cytosine or an analog thereof, thymine or an analog thereof, and uracil or an analog thereof.

6. The sequencing method according to claim 4, wherein in step (ii), B is selected from adenine or an analog thereof, cytosine or an analog thereof, thymine or an analog thereof, and uracil or an analog thereof, and the nucleotides or the analogs thereof have a final concentration of 500-1000 nmol/L, respectively; and B is guanine or an analog thereof, and the nucleotide or the analog thereof has a final concentration of 125-500 nmol/L.

7. The sequencing method according to any one of claims 1 to 6, wherein the optically detectable labels carried by the different nucleotides or analogs thereof are different.

8. The sequencing method according to claim 7, wherein the optically detectable label comprises one or more of Atto532, CY5, IF700, ROX, a rhodamine dye, a cyanine dye, a coumarin dye, or a phycoerythrin.

9. The sequencing method according to claim 7, wherein the nucleotide or the analog thereof is an adenine nucleotide or an analog thereof, and the optically detectable label of the nucleotide or the analog thereof is Atto532; the nucleotide or the analog thereof is a guanine nucleotide or an analog thereof, and the optically detectable label of the nucleotide or the analog thereof is CY5; the nucleotide or the analog thereof is a cytosine nucleotide or an analog thereof, and the optically detectable label of the nucleotide or the analog thereof is IF700; the nucleotide or the analog thereof is a thymine nucleotide or an analog thereof, and the optically detectable label of the nucleotide or the analog thereof is ROX.

10. The sequencing method according to any one of claims 1 to 9, wherein in step (ii), the four types of nucleotides or analogs thereof carrying the optically detectable labels are bound to the nucleic acid template-primer complex by using a polymerase to achieve the single-base extension, wherein the polymerase is a DNA polymerase and/or RNA polymerase, and the polymerase has a final concentration of 0.005-0.02 mg/mL.

11. The sequencing method according to claim 10, wherein the polymerase is selected from one or more of 9°N enzyme, Pfu, KOD1, and MMS2.

12. The sequencing method according to any one of claims 1 to 11, wherein in step (ii), the polymerization reaction is performed at a pH of 8.0-9.0.

13. The sequencing method according to any one of claims 1 to 12, wherein the optically detectable label is linked to the nucleotide or analog thereof via the chemically cleavable group, and in step (iv), removing the optically detectable label on the extension product is achieved by using a cleavage reagent comprising an organic phosphine and a buffer.

14. The sequencing method according to claim 13, wherein removing the optically detectable label on the extension product is performed at a pH value of 8.0-10.5 and a temperature of 39-65 °C.

15. The sequencing method according to claim 13, wherein the organic phosphine has a concentration of 10-100 mmol/L; and/or the buffer has a concentration of 0.01-2.0 mol/L.

16. The sequencing method according to claim 13, wherein the organic phosphine is selected from tris(3-hydroxypropyl)phosphine, tris(2-carboxyethyl)phosphine, tris(hydroxymethyl)phosphine, triphenylphosphine, and tris(2-hydroxyethyl)phosphine.

17. The sequencing method according to any one of claims 1 to 16, wherein in step (iii), exciting the optically detectable label to generate the optical signal comprises performing exposure processing on the solid carrier, wherein the exposure processing lasts for 100-400 ms.

18. The sequencing method according to any one of claims 1 to 17, wherein the optically detectable labels comprise a first optically detectable label and a second optically detectable label, and excitation wavelengths of the first optically detectable label and the second optically detectable label differ by at least 50 nm.

19. The sequencing method according to claim 18, wherein the first optically detectable label is a red-light-excitable fluorophore, and the red-light-excitable fluorophore has an exposure time of 100-400 ms; the second optically detectable label is a green-light-excitable fluorophore, and the green-light-excitable fluorophore has an exposure time of 50-100 ms.
